# EUROPEAN PATENT APPLICATION

(11) **EP 2 784 163 A1**
(43) Date of publication of application: **01.10.2014**
(21) Application number: 13382108.2
(22) Date of filing: 25.03.2013
(51) Int. Cl.: C12Q 1/68, G01N 33/53

(54) **Methods for the prognosis and diagnosis of neurodegenerative diseases**

(71) Applicant: Centro De Investigación Biomédica En Red De Enfermedades Neurodegenerativas, 28031 Madrid (ES); Consejo Superior De Investigaciones Científicas, 28006 Madrid (ES); Fundación Cien, 28031 Madrid (ES)
(72) Inventor: Naranjo Orovio, José Ramón, 28049 Madrid (ES); Mellström, Britt, 28049 Madrid (ES); Rábano Gutiérrez del Arroyo, Alberto, 28031 Madrid (ES)
(74) Representative: ABG Patentes, S.L.

(57) **Abstract**

The present invention corresponds to the field of neurobiology and relates to methods for predicting the appearance of a neurodegenerative disease in a subject, for diagnosing the prodromic stage of a neurodegenerative disease in a subject, for predicting whether a subject diagnosed of a prodromic stage of a neurodegenerative disease will develop said neurodegenerative disease and for selecting a subject for a therapy for the prevention and/or treatment of a prodromic stage of a neurodegenerative disease or of a neurodegenerative disease, all of them based on the alteration of the expression levels of the DREAM-encoding gene in a tissue sample comprising lymphocytes or in a biofluid sample from said subject.

## Description

### FIELD OF THE INVENTION

The present invention belongs to the field of neurodegenerative disease development prediction and is related to methods useful in the prediction of the appearance of a neurodegenerative disease in a subject, in the diagnosis of the prodromic stage of a neurodegenerative disease, in the selection of a subject for a prevention treatment of a prodromic stage of a neurodegenerative disease, all of them comprising determining the expression levels of the gene encoding DREAM in a sample from said subject and comparing said expression level to a reference value.

### BACKGROUND OF THE INVENTION

Neurodegenerative diseases are characterized by the death of neurons in different regions of the nervous system and the subsequent functional deterioration of the affected parts. The most common and well-known examples of these pathologies are Alzheimer's and Parkinson's diseases, although there are others, such as Huntington's disease, Down syndrome, ataxias, amyotrophic lateral sclerosis, etc., which also belong to the same clinical group. Neurodegenerative diseases have an enormous impact on the lives of the individuals suffering them and their families and on society as a whole as well.

Alzheimer disease (AD) is characterized by adult-onset progressive dementia associated with cerebral cortical atrophy, beta-amyloid plaque formation, and intraneuronal neurofibrillary tangles. AD typically begins with subtle memory failure that becomes more severe and is eventually incapacitating. Other common findings include confusion, poor judgment, language disturbance, agitation, withdrawal, hallucinations, seizures, Parkinsonian features, increased muscle tone, myoclonus, incontinence and autism.

Alzheimer's disease (AD) is a neurodegenerative disorder with high incidence and devastating personal and social consequence. Despite being first described long time ago, nowadays there is no effective diagnostic method for its early onset, with the consequence of delayed beginning of the treatment and reduced probability of success.

Different methods and strategies for prediction of the onset and development of a neurodegenerative disease in a subject have been developed in the art. It has been described that RNA plays an important role in a number of neurodegenerative diseases, and a theoretical framework has been developed to analyze ribonucleoprotein interactions linked to diseases such as Alzheimer's disease and Parkinson's disease (Cirillo D et al. 2013 RNA, 19: 129-140). US2012/0252062A1 describes a method for the diagnosis and/or the prediction of the development of a neurodegenerative disease comprising isolation of white blood cells from a blood sample of the subject, culture of the cells and evaluation of the colonies formed. Olfactory disfunction has also been proposed as a predictor of the risk of development of Alzheimer's disease or Parkinson's disease (Alberts MW et al. 2006, Curr. Neurol. Neurosci. Reports 6: 379-386). Similarly, depression and anxiety have been reported to be independently predictive of conversion to AD in patients with mild cognitive impairment (MCI) (Gallaher D et al. 2011, Int. J. Geriatr. Psych. 26: 166-172). Combination of Aβ42 concentrations and hippocampal volumes has been suggested to best predict mild MCI progression to AD (Prestia A et al. 2013, Alzheimers Dement. doi: 10.1016/j.jalz.2012.09.016).

Despite the efforts made to date, there still exists a long-felt and continuing need in the art for reliable methods for predicting the onset of AD, in particular methods for the prediction of the early onset of AD, as well as the diagnosis of the prodromic stage of AD and for the identification of those subjects identified as suffering the prodromic stage of the disease which will develop the disease.

### SUMMARY OF THE INVENTION

In a first aspect, the invention relates to an *in vitro* method for predicting the appearance of a neurodegenerative disease in a subject comprising:
(i) determining the expression levels of the DREAM-encoding gene in a sample from said subject, wherein said sample is not a sample of a solid tissue of the central nervous system, and
(ii) comparing the expression levels obtained in (i) to a reference value,
wherein a decrease in the expression levels of the DREAM-encoding gene with respect to a reference value is indicative of a high probability of appearance of a neurodegenerative disease in said subject.

In a second aspect, the invention relates to an *in vitro* method for the diagnosis of the prodromic stage of a neurodegenerative disease in a subject comprising:
(i) determining the expression levels of the DREAM-encoding gene in a sample from said subject, wherein said sample is not a sample of a solid tissue of the central nervous system, and
(ii) comparing the expression level obtained in (i) to a reference value,
wherein a decrease in the expression levels of the DREAM-encoding gene with respect to a reference value is indicative of a prodromic stage of a neurodegenerative disease in said subject.

In another aspect, the invention relates to an *in vitro* method for predicting whether a subject diagnosed with a prodromic stage of a neurodegenerative disease will develop said neurodegenerative disease comprising:
(i) determining the expression levels of the DREAM-encoding gene in a sample from said subject, wherein said sample is not sample from a solid tissue of the central nervous system, and
(ii) comparing the expression level obtained in (i) to a reference value,
   wherein a decrease in the expression levels of the DREAM-encoding gene with respect to a reference value is indicative of development of the neurodegenerative disease in a subject diagnosed of a prodromic stage of a neurodegenerative disease.

In another aspect, the invention relates to an *in vitro* method for the selection of a subject for a therapy for the prevention and/or treatment of a prodromic stage of a neurodegenerative disease or a therapy for the prevention and/or treatment of a neurodegenerative disease comprising
(i) determining the expression levels of the DREAM-encoding gene in a sample from said subject, wherein said sample is not a sample from a solid tissue of the central nervous system, and
(ii) comparing the expression level obtained in (i) to a reference value,
   wherein if the expression levels of the DREAM-encoding gene are decreased with respect to a reference value, then the subject is selected for said therapy.

In a further aspect, the invention relates to an *in vitro* method for determining whether a therapy for the prevention and/or treatment of a prodromic stage of a neurodegenerative disease in a subject suffering from a prodromic stage of a neurodegenerative disease or a therapy for the prevention and/or treatment of a neurodegenerative disease in a subject suffering from a neurodegenerative disease is effective comprising
(i) determining the expression levels of the DREAM-encoding gene in a sample from a subject treated with said therapy, wherein said sample is not a sample from a solid tissue of the central nervous system, and
(ii) comparing the expression level obtained in (i) to a reference value,
wherein if the expression levels of the DREAM-encoding gene are increased with respect to a reference value, then the therapy is effective.

In a last aspect, the invention relates to an anti-neurodegenerative therapy for use in the prevention and/or treatment of a prodromic stage of a neurodegenerative disease or for use in the prevention and/or treatment of a neurodegenerative disease in a subject, wherein the expression levels of the DREAM-encoding gene in a sample of said subject are decreased with respect to a reference value and wherein said sample is not a sample from a solid tissue of the central nervous system.

### DESCRIPTION OF THE FIGURES

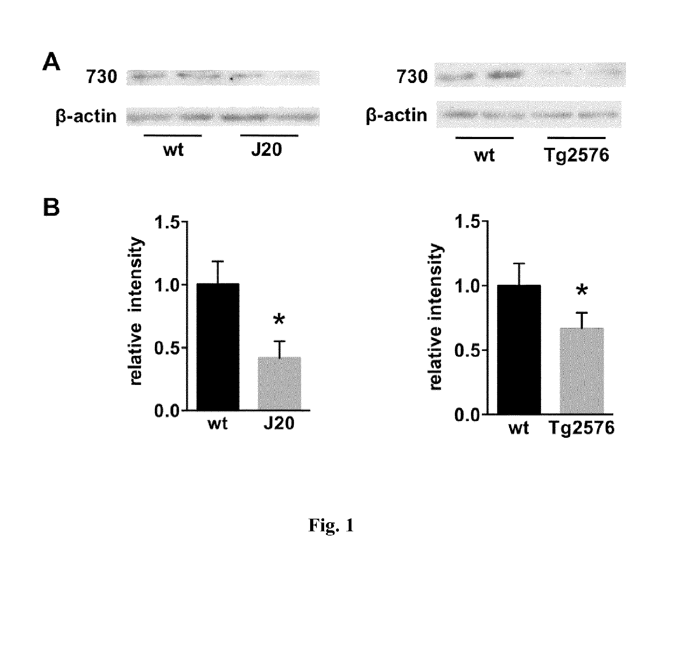

**Figure 1. (A)** Western blot analysis of DREAM (Ab 730) in thymus from 3-months old J20 and Tg2576 mice. **(B)** Histograms showing data from three experiments in duplicate, expressed as the ratio to β-actin levels and normalized with values from wild type (wt) mice. Data are presented as mean ± SEM (*, p < 0.05).

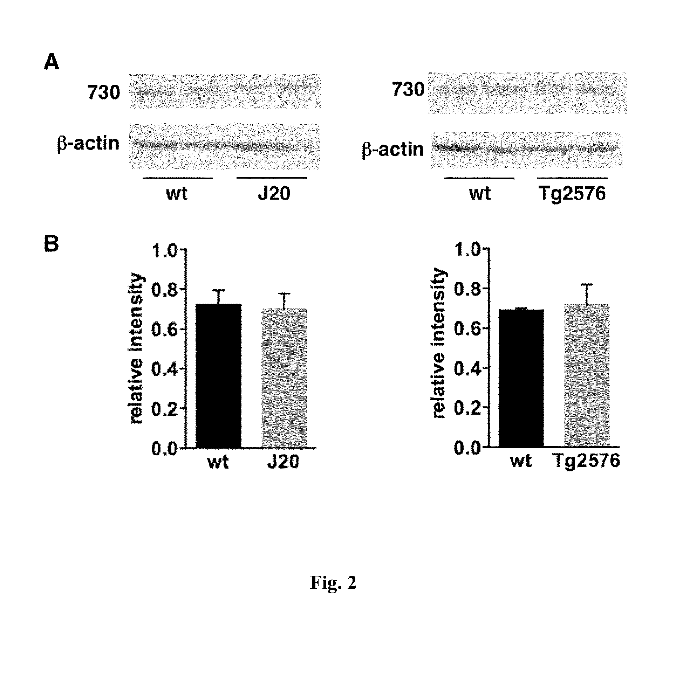

**Figure 2. (A)** Western blot analysis of DREAM (Ab 730) in testis from 3-months old J20 and Tg2576 mice. **(B)** Histograms showing data from three experiments in duplicate, expressed as the ratio to β -actin levels and normalized with values from wild type (wt) mice. Data are presented as mean ± SEM (*, p < 0.05).

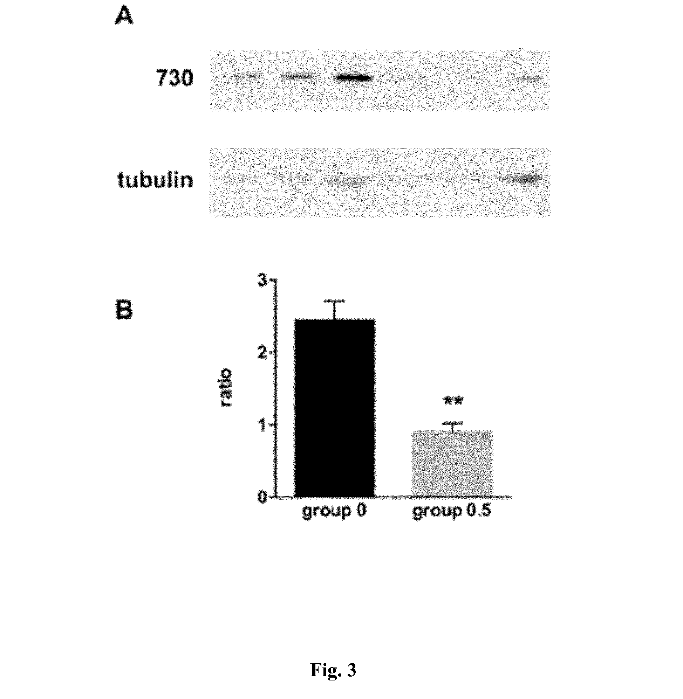

**Figure 3. (A)** Western blot analysis of DREAM (Ab 730) in PBLs whole cell extracts from ten > 70 year-old healthy volunteers; five from group 0 and five from group 0.5 with mild cognitive impairment. **(B)** Histograms showing data from the two groups, expressed as the ratio to tubulin levels. Data are presented as mean ± SEM (**, p < 0.01). This group of 20 volunteers belongs to a cohort that includes 940 individuals and is the base of a longitudinal study (The Vallecas Project) carried out by the Alzheimer Center, CIEN Foundation and Reina Sofia Foundation in Madrid. Upon written consent to participate in the study, a team of psychologists, psychiatrists and neurologists examines these volunteers. In parallel, blood and urine samples are taken and aliquots of serum and PBLs are prepared and stored in liquid nitrogen until analysis. The clinical and neuropsychological assessment includes different tests to evaluate cognition (mini mental state examination or MMSE, functional activities questionnaire or FAQ Pfeffer, Isaac test, Symbol Digit), motor function (Study on Cognition and Prognosis in the Elderly or SCOPE) and depressive symptoms (Yesavage scale). In addition, the volunteer is analyzed by MR neuroimaging using various sequences and techniques, including three-dimensional spoiled gradient-recalled echo (3D-SPGR), fluid-attenuated inversion recovery (FLAIR), ASL (arterial spin labeling) and functional NMR-Echo-Planar blood oxygenation level dependent (BOLD). Importantly, the medical evaluation and the amyloid determination are repeated once a year during a period of three years.

### DETAILED DESCRIPTION OF THE INVENTION

The authors of the present invention have established a functional link between reduced DREAM protein expression and Alzheimer's disease (AD). Moreover, they have identified DREAM as an early biomarker of clinical/diagnostic interest for the detection of AD in humans, since they were able to detect changes in DREAM levels in peripheral samples relevant for their clinical practice.

The authors have analyzed DREAM protein expression levels in the thymus and testis of two AD mouse models, namely J20 and Tg2576, showing no change in the expression levels of the protein in testis and reduced expression in thymus (see Example 1). Additionally, they have analyzed DREAM protein expression levels in human clinical samples of circulating peripheral blood lymphocytes, showing higher DREAM protein levels in those subjects without clinical symptoms of AD in comparison to subjects reporting sporadic failures of memory (see Example 2).

### Definitions

The term "Alzheimer's disease" or "senile dementia" or AD refers to a mental deterioration associated with specific degenerative brain disease that is characterized by senile plaques, neuritic tangles and progressive neuronal loss which manifests clinically in progressive memory deficits, confusion, behavioral problems, inability to care for oneself, gradual physical deterioration and, ultimately, death. Patients suffering Alzheimer's disease are identified using the NINCDS-ADRDA (National Institute of Neurological and Communicative Disorders and the Alzheimer's Disease and Related Disorders Association) criteria: Clinical Dementia Rating (CDR) = 1; Mini Mental State Examination (MMSE) between 16 and 24 points and Medial temporal atrophy (determined by Magnetic Resonance Imaging, MRI) >3 points in Scheltens scale.

The term "solid tissue from the central nervous system" or "CNS solid tissue" relates to any solid which can be obtained from the central nervous system of a subject and includes spinal cord tissue samples (including spinal cord grey and white matter tissue samples) and brain tissue samples (including tissue samples from cerebral cortex, the three layers of cerebellar cortex, the meninges, the connective tissue surrounding the components of the central nervous system, and choroid plexus). This term does not include CNS fluid samples, such as cerebrospinal fluid (CSF) or interstitial fluid (ISF). In the context of the present invention, the sample from a subject wherein the expression levels of DREAM-encoding gene are determined is not sample of a solid tissue of the central nervous system.

The term "diagnosis" as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As the person skilled in the art will understand, such a diagnosis may not be correct for 100% of the subject to diagnose, although preferred it is. The term, however, requires that a statistically significant part of the subjects can be identified as suffering from a disease, particularly a neurodegenerative disease in the context of the invention, or having a predisposition thereto. The skilled in the art may determine whether a party is statistically significant using different statistical evaluation tools well known, for example, by determination of confidence intervals, the p-value determination, Student's-test, the Mann-Whitney, etc. (see Dowdy and Wearden, 1983). Preferred confidence intervals are at least, 50%, at least 60%, at least 70%, at least 80%, at least 90% or at least 95%. The p-values are preferably, 0.05, 0.025, 0.001 or lower.

The term "DREAM" (Downstream Regulatory Element Antagonist Modulator, also known as calsenilin, potassium channel interacting protein-3, KChIP3, presenilin binding protein or CSEN, relates to a multifunctional Ca²⁺-binding protein belonging to calcium neuronal sensors superfamily, that binds specifically to DNA target sequences and that interacts with several nucleoproteins, having different functions depending on the subcellular compartment. In particular, the sequence of human DREAM protein is provided in the NCBI database entry NP_038462.1 (release of February 25^{th}, 2013), corresponding to the longer isoform or isoform 1 and entry NP_001030086.1 (release of February 25^{th}, 2013), corresponding to the shorter, unique N-terminus, isoform 2 precursor of the protein. In a preferred embodiment, the DREAM refers to isoform 1 of the DREAM protein. In another embodiment, DREAM refers to isoform 2 of the DREAM protein. In another embodiment, DREAM refers to the combination of isoforms 1 and 2 of the DREAM protein.

The term "DREAM-encoding gene" or "gene encoding DREAM" or "gene encoding DREAM protein" or similar terms relate to the gene the expression of which results in the DREAM protein and, particularly in humans, refers to the *KCNIP3* gene (Gene ID: 30818, NCBI database update of March 3^{rd}, 2013), located at chromosomal region 2q21.1. The mRNA sequence derived from human gene is provided in the NCBI database entry NM_013434.4 (release of February 25^{th}, 2013), which encodes the longer isoform 1 of the protein and NM_001034914.1 (release of February 25^{th}, 2013), corresponding to an alternate in-frame segment in the 5' coding region which encodes a shorter isoform 2 of the protein.

The term "expression level of a gene", as used herein, refers to the measurable quantity of gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene. In the context of the present invention, the expression level of the DREAM-encoding gene can be determined by measuring the levels of mRNA encoded by said gene, or by measuring the levels of the protein encoded by said gene, i.e. DREAM protein or of variants thereof. DREAM protein variants include all the physiologically relevant post-translational chemical modifications forms of the protein, for example, glycosylation, phosphorylation, acetylation, etc., provided that the functionality of the protein is maintained. Said term encompasses the DREAM protein of any mammal species, including but not being limited to domestic and farm animals (cows, horses, pigs, sheep, goats, dogs, cats or rodents), primates and humans. Preferably, the DREAM protein is a human protein.

The term "Huntington's disease" (HD) relates to a neurodegenerative genetic disorder that affects muscle coordination and leads to cognitive decline and psychiatric problems. It is caused by an autosomal dominant mutation in either of an individual's two copies of *Huntingtin* gene.

The term "lymphocyte" relates to a cell of a vertebrate immune system and comprises T lymphocytes (T cells), B lymphocytes (B cells) and natural killer (NK) cells. In a particular embodiment of the invention, the lymphocytes are T lymphocytes.

The term "mild cognitive impairment" or MCI is related to a transitional stage of cognitive impairment between normal aging and early Alzheimer's disease. Patients are usually identified as having MCI if they fulfill the Mayo Clinic criteria (Clinical Dementia Rating, CDR = 0.5, they show a medial temporal atrophy (determined by Magnetic Resonance Imaging, MRI) which is higher than 3 points in Scheltens scale, they show a pattern of parietal and/or temporal hypometabolism in Positron Emission Tomography with 18-fluorodeoxyglucose (PET-FDG) (suggestive of AD).

The term "neurodegeneration" relates to the process of progressive loss of structure or function of neurons, including death of neurons.

The expression "neurodegeneration related to Down's syndrome" is related to age-related neurodegeneration and memory loss occurring in Down syndrome, a condition where a complete or segmental chromosome 21 trisomy causes variable intellectual disability, and progressive memory loss and neurodegeneration with age.

The term "neurodegenerative disease" refers to a condition or disorder in which neuronal cells are lost due to cell death bringing about a deterioration of cognitive functions or result in damage, dysfunction, or complications that may be characterized by neurological, neurodegenerative, physiological, psychological, or behavioral aberrations. Neurodegenerative diseases include, without being limited to, age-related macular degeneration, Creutzfeldt-Jakob disease, Alzheimer's Disease, radiotherapy induced dementia, axon injury, acute cortical spreading depression, alpha-synucleinopathies, brain ischemia, Huntington's disease, permanent focal cerebral ischemia, peripheral nerve regeneration, post-status epileptics model, spinal cord injury, sporadic amyotrophic lateral sclerosis and transmissible spongiform encephalopathy. In a particular embodiment, the neurodegenerative diseases include, but are not limited to, Alzheimer's disease, Huntington's disease, Parkinson's disease and neurodegeneration related to Down's syndrome. In a more particular embodiment, the neurodegenerative disease is Alzheimer's disease.

The term "Parkinson's disease" relates to a degenerative disorder of the central nervous system. The characteristic motor symptoms of this disease result from the death of dopamine-generating cells in the substantia nigra, a region of the midbrain. Early in the course of the disease, the most obvious symptoms are movement-related; these include shaking, rigidity, slowness of movement and difficulty with walking and gait. Later, cognitive and behavioral problems may arise, with dementia commonly occurring in the advanced stages of the disease. Other symptoms include sensory, sleep and emotional problems.

The term "prodromic stage" is used in the present invention in the context of a disease and is related to the stage characterized by the initial symptoms preceding the development of a disease, more particularly a neurodegenerative disease. In a preferred embodiment, the prodromic stage of a neurodegenerative disease according to the invention comprises mild cognitive impairment (MCI).

The term "reference value", as used herein, refers to a laboratory value used as a reference for values/data obtained by laboratory examinations of subjects or samples collected from subjects. Suitable reference values are indicated in the context of each of the methods of the invention.

The term "sample" or "biological sample" according to the invention relates to any biological tissue, different from a solid sample of the central nervous system as well as to any biofluid. In a particular embodiment, the sample that is obtained from a subject is not a sample from a solid tissue of the central nervous system. In another embodiment, the sample is not a testis sample. In another embodiment, the sample comprises lymphocytes. In a particular alternative embodiment, the sample is a biofluid. In a particular embodiment, the biofluid comprises lymphocytes. In an alternative particular embodiment, the biofluid does not comprise lymphocytes. In a preferred embodiment, the biofluid is selected from blood, particularly peripheral blood, serum or plasma. The blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Serum can be obtained from the complete blood sample and in the absence of anticoagulant by leaving the sample to settle for 10 minutes so that it coagulates and subsequently centrifuging it at 1,500 rpm for 10 minutes for the purpose of separating the cells (precipitate) from the serum (supernatant). In a particular alternative embodiment, the sample is any tissue sample which is not from a solid tissue of the central nervous system. In another embodiment, the sample is a sample containing lymphocytes. In a preferred embodiment, the tissue sample is a thymus sample. Tissue samples can be obtained by any method known in the art including, without being limited to, surgical excision, aspiration or biopsy. In an alternative preferred embodiment, the sample is a biofluid, preferably selected from blood and serum.

The term "subject" relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age, sex or race.

The term "therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on the prodromic stage of a neurodegenerative disease or on the neurodegenerative disease or, in other words, to be useful to treat a prodromic stage of a neurodegenerative disease or to treat a neurodegenerative disease. This term includes both therapeutic treatment and prophylactic or preventative measures, in which the object is to prevent or stop (reduce) an undesired physiological change or disorder, such as, neurodegenerative disorders. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival in comparison with the expected survival if treatment is not received. Those subjects needing treatment include those subjects already suffering the condition or disorder, as well as those with the tendency to suffer the condition or disorder or those in which the condition or disorder must be prevented.

The expression "therapy for the prevention and/or treatment of a neurodegenerative disease" in the present invention, relates to any therapy or compound aimed at preventing or remediating a neurodegenerative disease including, without being limited to, beta-amyloid plaque inhibitors, amyloid synthesis inhibitors, beta-secretase inhibitors, MAO-B inhibitors, acetylcholinesterase inhibitors or NMDA receptor antagonists. Examples include, without being limited to:
- antibodies such as bapineuzumab, a monoclonal antibody (mAb) that targets the amino terminus of β-amyloid and is aimed at slowing or even halting AD progression (in Phase III trials by Janssen); antisenilin®, which is similar to bapineuzumab but targets either end of β-amyloid (in Phase III analysis by Intellect Neurosciences); gammagard, a naturally occurring plasma-derived immunoglobulin for moderate-stage AD patients (in Phase II trials, already approved for immunodeficiency diseases) or gantenerumab, a fully human mAb (in Phase II trials by Roche),
- inhibitors of monoamine oxidase-B (MAO-B), such as the small molecule inhibitor of MAO-B (EVT-302) which is in Phase II trials by Roche,
- inhibitors of beta-secretase (BACE), which is a key enzyme in the production of β-amyloid peptides, such as memantine (Axura and Akatinol by Merz, Namenda by Forest, Ebixa and Abixa by Lundbeck and Memox by Unipharm); the small molecule inhibitor of BACE which is in Phase I trials by Roche; CTS-21166 (in Phase I trials in March 2008), MK-8931 (in Phase I trials in April 2012 by Merck), and MK-8931 (in Phase II/II trials in December 2012 by Merck).
- acetylcholinesterase inhibitors such as Aricept (donepezil HC1, approved to treat mild, moderate and severe AD), tacrine (Cognex®), rivastigmine (Exelon) and galantamine (Razadyne, Reminyl).

The expression "therapy for the prevention and/or treatment of a prodromic stage of a neurodegenerative disease" relates to any therapy or compound aimed at preventing or remediating a prodromic stage of a neurodegenerative disease, comprising mild cognitive impairment (MCI). There is no current treatment for MCI approved by the FDA or Canadian government regulators, but in Europe medications such as Ginkgo biloba and Hydergine® (ergoloid mesylate) have been approved for memory impairment. Symptomatic therapies proposed for MCI include, without being limited to, cholinesterase inhibitors, Ginkgo biloba, nootropic medications (such as phosphatidyl serine, acetyl-L-carnitine, phosphatidylcholine or pyracetam) and memory stimulants (such as ampakines, NMDA receptor modulators and CREB modulators). Preventive therapies proposed for MCI include, without being limited to, anti-oxidants (such as vitamin E, selegiline or vitamin C), homocysteine, omega fatty acids, cholinesterase inhibitors, anti-inflammatory agents, estrogen, statins, anti-amyloid therapies (such as beta- and gamma-secretase inhibitors, or GAG mimetics), and therapies for vascular risk factors.

### Method for predicting the appearance of a neurodegenerative disease in a subject (first method of the invention)

The authors of the present invention have analyzed DREAM protein expression levels in the thymus and testis of two mouse models for Alzheimer's disease (J20 and Tg2576 mice). They observed no change in the expression levels of the protein in testis, altogether with reduced expression of the protein in the thymus (see Example 1). These results suggest a localized modification of DREAM expression protein in AD detection.

Thus, in a first aspect, the present invention is related to an *in vitro* method for predicting the appearance of a neurodegenerative disease in a subject (*first method of the invention*) comprising:
(i) determining the expression levels of the DREAM-encoding gene in a sample from said subject, wherein said sample is not a sample of a solid tissue of the central nervous system, and
(ii) comparing the expression levels obtained in (i) to a reference value,
wherein a decrease in the expression levels of the DREAM-encoding gene with respect to a reference value is indicative of a high probability of appearance of a neurodegenerative disease in said subject.

Accordingly, in a first step of the first method of the invention, the expression levels of the DREAM-encoding gene are determined in a sample that is not a central nervous system tissue solid sample from a subject whose appearance of a neurodegenerative disease is to be predicted.

The sample from a subject according to the invention is a tissue sample which is not from a solid tissue of the central nervous system. In one embodint, the sample is a biofluid sample. In a particular embodiment, the tissue sample which is not from a solid tissue of the central nervous system is a sample which comprises lymphocytes, more particularly T lymphocytes. The sample can be obtained by any conventional method depending on the sample to be analyzed. In an alternative particular embodiment, the sample is a body fluid sample, e.g., a sample comprising lymphocytes from the subject such as a blood sample. In an alternative particular embodiment, the sample is a body fluid sample which does not comprise lymphocytes, such as a serum sample or a plasma sample. Any tissue sample, different from a sample of a solid tissue of the central nervous system and which comprises lymphocytes, particularly T lymphocytes, from a subject may be used as well. Examples of tissue samples that may be used include, but are not limited to, thymus and bone marrow. The tissue sample can be obtained by a variety of procedures including, but not limited to, surgical excision, aspiration or biopsy. The tissue may be fresh or frozen. In one embodiment, the tissue sample is fixed and embedded in paraffin or the like. The tissue sample may be fixed (i.e. preserved) by conventional methodology [See e.g., "Manual of Histological Staining Method of the Armed Forces Institute of Pathology," 3rd edition (1960) Lee G. Luna, HT (ASCP) Editor, The Blakston Division McGraw-Hill Book Company, New York; The Armed Forces Institute of Pathology Advanced Laboratory Methods in Histology and Pathology (1994) Ulreka V. Mikel, Editor, Armed Forces Institute of Pathology, American Registry of Pathology, Washington, D.C]. By way of example, neutral buffered formalin, Bouin's or paraformaldehyde, may be used to fix a tissue sample. In a preferred embodiment, the sample comprising lymphocytes wherein the expression levels of the DREAM-encoding gene are to be determined is a blood sample, more preferably a peripheral blood sample, or a thymus sample. In another preferred embodiment, the fluid sample not comprising lymphocytes wherein the expression levels of the DREAM-encoding gene are to be determined is a serum sample.

Before analyzing the sample, it will often be desirable to perform one or more preparation operations upon said sample aimed at separating the molecule to be determined (protein or mRNA) from other molecules found in the sample. Typically, these sample preparation operations include manipulations such as concentration, suspension, extraction of intracellular material, e.g., nucleic acids from tissue/whole cell samples and the like, amplification of nucleic acids, fragmentation, transcription, labeling and/or extension reactions. Nucleic acids, especially RNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. These methods are typically well known by a person skilled in the art. Extraction of proteins in general involves one or more steps of removal of or separation from contaminating nucleic acids, phages and/or viruses, other proteins and/or other biological macromolecules. Methods of protein extraction are well known in the art (see, for example "Protein Methods", D. M. Bollag et al., 2nd Ed., 1996, Wiley-Liss; "Protein Purification Methods: A Practical Approach", E. L. Harris and S. Angal (Eds.), 1989; "Protein Purification Techniques: A Practical Approach", S. Roe, 2nd Ed., 2001, Oxford University Press; "Principles and Reactions of Protein Extraction, Purification, and Characterization", H. Ahmed, 2005, CRC Press: Boca Raton, Fla.). In some embodiments, after a protein extract has been obtained, the protein concentration of the extract is standardized to a value being the same as that of the control sample in order to allow signals of the protein markers to be compared and/or quantitated. Such standardization can be made using photometric or spectrometric methods or gel electrophoresis.

In the context of the present invention, the expression levels of the DREAM-encoding gene in a sample of a subject can be determined by measuring the levels of mRNA encoded by said gene, or by measuring the levels of the protein encoded by said gene, i.e. DREAM protein or of functionally equivalent variants thereof.

In a particular preferred embodiment of the invention, the expression levels of the DREAM-encoding gene are determined by measuring the expression levels of DREAM protein or of functionally equivalent variants thereof.

As it is used herein, "functionally equivalent variant of DREAM protein" is understood as any molecule sharing with DREAM at least the specific binding to DRE sites in the DNA and repressing transcription in a Ca²⁺-dependent manner associated with DREAM protein, both *in vitro* and *in vivo,* and having a minimal identity in the amino acid sequence. Functionally equivalent variants of DREAM protein are related to: i) variants of the protein in which one or more of the amino acid residues are substituted by a conserved or non-conserved amino acid residue (preferably a conserved amino acid residue), wherein such substituted amino acid residue may or may not be one encoded by the genetic code, or ii) variants comprising an insertion or a deletion of one or more amino acids and having the same function as the protein. Methods for determining whether a protein binds specific DNA elements are known by the skilled person and include, without being limited to, chromatin immunoprecipitation (ChIP) assays, electrophoretic mobility shift assays (EMSA), DNA pull-down assays, reporter assays, microplate capture and detection assays, and DNase I footprinting assays.

Variants comprise all those variants of human DREAM protein mentioned above which occur naturally in other species, i.e., DREAM orthologs. Said natural variants include but are not limited to DREAM rat protein (NCBI database entry NP_115851.1 for *Rattus norvegicus,* 256 aa, release as of 25-FEB-2013), DREAM mouse protein (NP_062763.2 for *Mus musculus,* 256 aa, release as of 9-FEB-2013), DREAM dog protein (XP_003639644.1, predicted sequence for *Canis lupus,* 256 aa, release as of 2-DEC-2011), DREAM chimpanzee protein (XP_515619, predicted sequence for *Pan troglodytes,* 256, aa, release as of 25-OCT-2012) and DREAM rhesus monkey protein (NP_001244793 for *Macaca mulatta,* 256 aa, release as of 15-APR-2012). The natural variants of DREAM can also be derived from said sequences by means of insertion, substitution or deletion of one or more amino acids and include natural alleles, variants resulting from alternative processing and secreted and truncated forms occurring naturally.

Additionally, the functionally equivalent variants of DREAM contemplated in the first aspect of the invention, include polypeptides showing at least 60%, 65%, 70%, 72%, 74%, 76%, 78%, 80%, 90%, 95%, 97%, 99% similarity or identity with the different natural variants of DREAM mentioned above. The degree of identity between two polypeptides is determined using algorithms implemented in a computer and methods which are widely known by the persons skilled in the art. The identity between two amino acid sequences is preferably determined using the BLASTP algorithm (BLAST Manual, Altschul, S. *et al*., NCBI NLM NIH Bethesda, Md. 20894, Altschul, S., et al., 1990, Mol. Biol. 215:403-410).

In general, protein expression levels are determined by contacting a biological sample isolated from a subject with binding agents for one or more of the protein markers; detecting, in the sample, the levels of polypeptides that bind to the binding agents; and comparing the levels of polypeptides in the sample with the levels of polypeptides in a control sample. As used herein, the term "binding agent" refers to an entity such as a polypeptide or antibody that specifically binds to an inventive protein marker. An entity "specifically binds" to a polypeptide if it reacts/interacts at a detectable level with the polypeptide but does not react/interact detectably with peptides containing unrelated sequences or sequences of different polypeptides.

In some embodiments, the binding agent is an antibody specific for DREAM. Suitable antibodies include antibodies specific for the isoform 1 of DREAM (i.e. antibodies which do not cross-react with the isoform 2), antibodies specific for the isoform 2 (i.e. antibodies which do not cross-react with isoform 1) and antibodies which bind both DREAM isoforms. Antibodies for use in the methods of the present invention include monoclonal and polyclonal antibodies, immunologically active fragments (e.g., Fab or (Fab)2 fragments), antibody heavy chains, humanized antibodies, antibody light chains, and chimeric antibodies. Antibodies, including monoclonal and polyclonal antibodies, fragments and chimeras, may be prepared using methods known in the art (see, for example, R. G. Mage and E. Lamoyi, in "Monoclonal Antibody Production Techniques and Applications", 1987, Marcel Dekker, Inc.: New York, pp. 79-97; G. Kohler and C. Milstein, Nature, 1975, 256: 495-497; D. Kozbor et al., J. Immunol. Methods, 1985, 81: 31-42; and R. J. Cote et al., Proc. Natl. Acad. Sci. 1983, 80: 2026-203; R. A. Lerner, Nature, 1982, 299: 593-596; A. C. Nairn et al., Nature, 1982, 299: 734-736; A. J. Czernik et al., Methods Enzymol. 1991, 201: 264-283; A. J. Czernik et al., Neuromethods: Regulatory Protein Modification: Techniques and Protocols, 1997, 30: 219-250; A. J. Czernik et al., Neuroprotocols, 1995, 6: 56-61; H. Zhang et al., J. Biol. Chem. 2002, 277: 39379-39387; S. L. Morrison et al., Proc. Natl. Acad. Sci., 1984, 81: 6851-6855; M. S, Neuberger et al., Nature, 1984, 312: 604-608; S. Takeda et al., Nature, 1985, 314: 452-454). Antibodies to be used in the methods of the invention can be purified by methods well known in the art (see, for example, S. A. Minden, "Monoclonal Antibody Purification", 1996, IBC Biomedical Library Series: Southbridge, Mass.). For example, antibodies can be affinity-purified by passage over a column to which a protein marker or fragment thereof is bound. The bound antibodies can then be eluted from the column using a buffer with a high salt concentration.

Antibodies specific for DREAM protein are commercially available including, without limitation, the polyclonal anti-human DREAM antibody raised in rabbit (FL-214, sc-9142) from Santa Cruz Biotechnology, the monoclonal anti-human DREAM antibody raised in mouse (A-9, sc-166916) from Santa Cruz Biotechnology, the polyclonal anti-human DREAM antibody raised in goat (K-17, sc-9309) from Santa Cruz Biotechnology, the polyclonal anti-human DREAM antibody raised in rabbit (PA1-16863) from Pierce Antibodies, the monoclonal anti-human DREAM antibody raised in mouse (clone 40A5 | 05-756) from Merck Millipore, the polyclonal anti-human DREAM antibody raised in rabbit (LS-B249) from LifeSpan Biosciences or the polyclonal anti-human DREAM antibody raised in rabbit (NB600-211) by Novus Biologicals.

In certain embodiments, the binding agent is directly or indirectly labeled with a detectable moiety. The role of a detectable agent is to facilitate the detection step of the diagnostic method by allowing visualization of the complex formed by binding of the binding agent to the protein marker (or analog or fragment thereof). Preferably, the detectable agent is selected such that it generates a signal which can be measured and whose intensity is related (preferably proportional) to the amount of protein marker present in the sample being analyzed. Methods for labeling biological molecules such as polypeptides and antibodies are well-known in the art (see, for example, "Affinity Techniques. Enzyme Purification: Part B", Methods in Enzymol., 1974, Vol. 34, W. B. Jakoby and M. Wilneck (Eds.), Academic Press: New York, N.Y.; and M. Wilchek and E. A. Bayer, Anal. Biochem., 1988, 171: 1-32).

Any of a wide variety of detectable agents can be used in the practice of the present invention. Suitable detectable agents include, but are not limited to: various ligands, radionuclides, fluorescent dyes, chemiluminescent agents, microparticles (such as, for example, quantum dots, nanocrystals, phosphors and the like), enzymes (such as, for example, those used in an ELISA, i.e., horseradish peroxidase, beta-galactosidase, luciferase, alkaline phosphatase), colorimetric labels, magnetic labels, and biotin, dioxigenin or other haptens and proteins for which antisera or monoclonal antibodies are available. Additional examples of detectable moieties or agents are provided in the Detectable Moieties section.

In certain embodiments, the binding agents (e.g., antibodies) may be immobilized on a carrier or support (e.g., a bead, a magnetic particle, a latex particle, a microtiter plate well, a cuvette, or other reaction vessel). Examples of suitable carrier or support materials include agarose, cellulose, nitrocellulose, dextran, Sephadex, Sepharose, liposomes, carboxymethyl cellulose, polyacrylamides, polystyrene, gabbros, filter paper, magnetite, ion-exchange resin, plastic film, plastic tube, glass, polyamine-methyl vinyl-ether-maleic acid copolymer, amino acid copolymer, ethylene-maleic acid copolymer, nylon, silk, and the like. Binding agents may be indirectly immobilized using second binding agents specific for the first binding agents (e.g., mouse antibodies specific for the protein markers may be immobilized using sheep anti-mouse IgG Fc fragment specific antibody coated on the carrier or support).

Protein expression levels in the diagnostic methods of the present invention may be determined using immunoassays. Examples of such assays include, but are not limited to, immunohistochemistry, time resolved fluorescence immunoassays (TR-FIA), radioimmunoassays, enzyme immunoassays (e.g., ELISA), immunofluorescence immunoprecipitation, latex agglutination, hemagglutination, which are conventional methods well-known in the art. As will be appreciated by one skilled in the art, an immunoassay may be competitive or non-competitive. Methods of detection and quantification of the signal generated by the complex formed by binding of the binding agent with the protein marker will depend on the nature of the assay and of the detectable moiety (e.g., fluorescent moiety).

In some embodiments, immunohistochemistry (IHC) is used to practice the methods of the invention. As used herein, immunohistochemistry refers to the process of localizing antigens (e.g., proteins) in cells of a tissue section based on the principle of specific antibody and antigen interactions. Typically, immunohistochemistry is performed on tissue slices. Depending on the purpose and the thickness of the sample, either thin (about 4-40 gm) slices are used, or if the tissue is not very thick and is penetrable, it may be used whole. Tissue slicing is usually accomplished through the use of a microtome, and slices are mounted on slides. In some embodiments, slices are used without mount. Such procedure is also known as "free-floating IHC." Visualising an antibody-antigen interaction can be accomplished in a number of ways. In some emboidments, an antibody is conjugated to an enzyme, such as peroxidase, that can catalyse a colour-producing reaction (see immunoperoxidase staining section) In some embodiments, the antibody can also be tagged to a fluorophore, such as fluorescein, rhodamine, DyLight Fluor, or Alexa Fluor (see Detectable Moiety section).

In some embodiments, the protein expression levels may be determined using mass spectrometry based methods or image (including use of labeled ligand) based methods known in the art for the detection of proteins. Other suitable methods include proteomics-based methods. Proteomics, which studies the global changes of protein expression in a sample, can include the following steps: (1) separation of individual proteins in a sample by electrophoresis (1-D PAGE), (2) identification of individual proteins recovered from the gel (e.g., by mass spectrometry or N-terminal sequencing), and (3) analysis of the data using bioinformatics.

On the other hand, the determination of the levels of protein encoded by *DREAM* gene can be carried out by constructing a tissue microarray (TMA) containing the subject samples assembled, and determining the expression levels of each corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in the sample under analysis and the specific cut-off for the marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

In another embodiment, the expression levels of the DREAM-encoding gene are determined by measuring the levels of the mRNA encoded by said gene. In order to measure the mRNA levels of DREAM-encoding gene, the biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

In a particular embodiment of the invention, the mRNA is obtained from a blood sample or from a thymus sample from a subject, preferably from a peripheral blood sample.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor.

Determination of the levels of mRNA of DREAM-encoding gene can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the levels of the above genes can also be carried out by Fluorescence In Situ Hybridization, including variants such as Flow-FISH, qFiSH and double fusion FISH (D-FISH) as described in WO2010030818, Femino et al. (Science, 1998, 280:585-590), Levsky et al. (Science, 2002, 297:836-840) or Raj et al. (PLoS Biology, 2006, 4:e309). The levels of the mRNA of the different genes can also be determined by nucleic acid sequence based amplification (NASBA) technology.

In a preferred embodiment, the gene mRNA expression levels are determined by reverse transcription polymerase chain reaction (RT-PCR). The detection can be carried out in individual samples or in tissue microarrays.

Thus, in a particular embodiment, the mRNA expression levels of DREAM-encoding gene are determined by quantitative PCR, preferably, Real-Time PCR. The detection can be carried out in individual samples or in microarrays.

In order to normalize the values of mRNA expression among the different samples, it is possible to compare the expression levels of the mRNA of interest in the test samples with the expression of a control RNA. A "control RNA" as used herein, relates to RNA whose expression levels does not change or changes only in limited amounts in samples under analysis with respect to control samples. Preferably, the control RNA is mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, IPO8 HPRT, GAPDH, PSMB4, tubulin and β-actin. In a preferred embodiment, the control RNA is GAPDH, IPO8, HPRT, β-actin, 18-S ribosomal protein or PSMB4 mRNA.

In one embodiment relative gene expression quantification is calculated according to the comparative threshold cycle (Ct) method using GAPDH, IPO8, HPRT, β-actin or PSMB4 as an endogenous control and commercial RNA controls as calibrators. Final results are determined according to the formula 2-(ΔCt sample-ΔCt calibrator), where ΔCT values of the calibrator and sample are determined by subtracting the Ct value of the target gene from the value of the control gene.

In a second step of the first method of the invention, the expression levels of the DREAM-encoding gene in a sample from a subject whose appearance of a neurodegenerative disease is to be predicted are compared to a reference value, wherein a decrease in the expression levels of the DREAM-encoding gene with respect to a reference value is indicative of a high probability of appearance of a neurodegenerative disease in said subject.

In the context of the first method of the invention, the expression "high probability of appearance of a neurodegenerative disease" is understood to mean the situation where the subject shows at least 5%, at least 10%, at least 20%, at least 30%, at least 40%, at least 50%, at least 60%, at least 70%, at least 80%, at least 90% or at least 100% probabilities of developing or suffering a neurodegenerative disease over time.

The reference value or reference level according to the methods of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a particular embodiment of the first method of the invention, the reference value is the expression level of the gene of interest, i.e. DREAM-encoding gene, in a healthy subject.-Particularly, the reference value according to the first method of the invention is the expression level of the DREAM-encoding gene of interest in a subject not diagnosed with a neurodegenerative disease or the expression level of the DREAM-encoding gene in a subject not diagnosed with a prodromic stage of a neurodegenerative disease. In another embodiment, the reference value according to the first method of the invention is an average value obtained from a pool of healthy subjects, particularly from a pool of subjects not diagnosed with a neurodegenerative disease or from a pool of subjects not diagnosed with a prodromic stage of a neurodegenerative disease. Said reference value sample is typically obtained by combining equal amounts of samples from a subject population.

In another embodiment, the expression levels are normalized expression levels. The term "normalized" expression level as used herein refers to an expression level of a gene relative to the expression level of a single reference gene, or a particular set of reference genes. Suitable reference genes include, without limitation, β-2-microglobulin, ubiquitin, ribosomal protein 18S, cyclophilin A, transferrin receptor, actin, GAPDH, tyrosine 3-monooxygenase/tryptophan 5-monooxygenase activation protein (YWHAZ), HPRT, and IPO8

Once the reference value of DREAM-encoding gene expression is established, the expression levels of the DREAM-encoding gene expressed in a sample from a subject can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

Thus, according to the predicting method of the invention, a decrease in the expression levels of the DREAM-encoding gene with respect to the reference value is indicative of a high probability of appearance of a neurodegenerative disease in said subject.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test as described in Dowdy and Wearden, Statistics for Research, John Wiley & Sons, New York 1983. Suitable confidence intervals are at least 50%, at least 60%, at least 70%, at least 80%, at least 90%, at least 95%. p values are, preferably, 0.2, 0.1, 0.05. Thus, the term "high probability of appearance of a neurodegenerative disease" is understood to mean the situation where the subject shows at 5 %, at least 10%, at least 20%, at least 30%, at least 40%, at least 50%, at least 60%, at least 70%, at least 80%, at least 90%, at least 100% probabilities of developing or suffering a neurodegenerative disease over time.

In a particular embodiment of the invention, the subject whose appearance of a neurodegenerative disease is to be predicted is a human.

Neurodegenerative diseases the appearance of which is predicted by the method of the invention comprise Alzheimer's disease, Huntington's disease, Parkinson's disease and neurodegeneration related to Down's syndrome. In a preferred embodiment, the neurodegenerative disease the appearance of which is predicted by the method of the invention is Alzheimer's disease (AD).

### Method for the diagnosis of the prodromic stage of a neurodegenerative disease in a subject (second method of the invention)

The authors of the present invention have analyzed the expression levels of DREAM protein in human clinical samples of circulating peripheral blood lymphocytes (PBL), showing that DREAM protein levels are higher in those subjects without clinical symptoms of Alzheimer's disease in comparison to subjects reporting sporadic failures of memory (see Example 2).

Thus, in a second aspect, the present invention relates to an *in vitro* method for the diagnosis of the prodromic stage of a neurodegenerative disease in a subject (*second method of the invention*) comprising:
(i) determining the expression levels of the DREAM-encoding gene in a sample from said subject, wherein said sample is not a sample of a solid tissue of the central nervous system, and
(ii) comparing the expression level obtained in (i) to a reference value,
wherein a decrease in the expression levels of the DREAM-encoding gene with respect to a reference value is indicative of a prodromic stage of a neurodegenerative disease in said subject.

In the context of the invention, the prodromic stage of a neurodegenerative disease comprises mild cognitive impairment (MCI).

Thus, in a first step of the second method of the invention, the expression levels of the DREAM-encoding gene are determined in a sample from a subject who is subjected to diagnosis. The sample from a subject to be diagnosed is a tissue sample which is not a sample of a solid tissue of the central nervous system. In another embodiment, the sample is a biofluid sample. In a particular embodiment, the sample from the subject to be diagnosed comprises lymphocytes, more particularly T lymphocytes. Preferred samples according to the invention comprise a thymus sample and a blood sample, preferably a peripheral blood sample. In an alternative particular embodiment, the sample is a biofluid not comprising lymphocytes, particularly a serum sample. In a particular embodiment, the subject is a human.

Methods for determining the expression levels of the DREAM-encoding gene include methods for determining the expression levels of the corresponding transcriptional product and methods for determining the expression levels of the corresponding translational product, and have been previously described in the context of the first method of the invention. In a particular embodiment of the second method of the invention, the expression levels are determined by measuring the levels of the DREAM protein or of variants thereof in a sample from a subject to be diagnosed according to the said second method.

In a second step of the second method of the invention, the expression levels of the DREAM-encoding gene determined in a sample of a subject who is subjected to diagnosis are compared to a reference value, wherein a decrease in the expression levels of the DREAM-encoding gene with respect to a reference value is indicative of a prodromic stage of a neurodegenerative disease in said subject.

Determination of the reference value has been described previously. In a particular embodiment, the reference value in the context of the second method of the invention is the expression level of the gene of interest, i.e. DREAM-encoding gene, in a healthy subject, more particularly in a subject not diagnosed with a neurodegenerative disease or in a subject not diagnosed with a prodromic stage of a neurodegenerative disease. In another embodiment, the reference value can correspond to an average value obtained from a pool of healthy subjects, particularly from a pool of subjects not diagnosed with a neurodegenerative disease or from a pool of subjects not diagnosed with a prodromic stage of a neurodegenerative disease. Said reference sample is typically obtained by combining equal amounts of samples from a subject population.

In a particular embodiment of the second method of the invention, the subject diagnosed with the prodromic stage of a neurodegenerative disease is a human. Particularly, the prodromic stage of a neurodegenerative disease under diagnosis in a subject comprises mild cognitive impairment (MCI).

Neurodegenerative diseases the prodromic stage of which is predicted by the second method of the invention comprise Alzheimer's disease, Huntington's disease, Parkinson's disease and neurodegeneration related to Down's syndrome. In a preferred embodiment, the neurodegenerative disease the prodromic stage of which is predicted by the method of the invention is Alzheimer's disease.

### Method for predicting whether a subject diagnosed of a prodromic stage of a neurodegenerative disease will develop said neurodegenerative disease (third method of the invention)

In a further aspect, the invention relates to a method for predicting whether a subject diagnosed of a prodromic stage of a neurodegenerative disease will develop said neurodegenerative disease (*third method of the invention*) comprising:
(i) determining the expression levels of the DREAM-encoding gene in a sample from said subject, wherein said sample is not a sample of a solid tissue of the central nervous system, and
(ii) comparing the expression level obtained in (i) to a reference value,
wherein a decrease in the expression levels of the DREAM-encoding gene with respect to a reference value is indicative of development of the neurodegenerative disease in a subject diagnosed of a prodromic stage of a neurodegenerative disease.

As previously mentioned, the prodromic stage of a neurodegenerative disease comprises mild cognitive impairment (MCI).

Thus, in a first step of the third method of the invention, the expression levels of the DREAM-encoding gene are determined in a sample from a subject who has been diagnosed of a prodromic stage of a neurodegenerative disease. The sample from a subject diagnosed of a prodromic stage of a neurodegenerative disease is a tissue sample different from a central nervous system solid tissue sample, or a biofluid sample. In a particular embodiment, the sample from the subject to be diagnosed comprises lymphocytes, more particularly T lymphocytes. Preferred samples according to the invention comprise a thymus sample and a blood sample, preferably a peripheral blood sample. In an alternative particular embodiment, the sample is a biofluid not comprising lymphocytes, particularly a serum sample.

In a particular embodiment, the subject is a human, more particularly a human who has been diagnosed of a prodromic stage of a neurodegenerative disease.

Methods for determining the expression levels of the DREAM-encoding gene include methods for determining the expression levels of the corresponding transcriptional product and methods for determining the expression levels of the corresponding translational product, and have been previously described in the context of the first method of the invention. In a particular embodiment, the expression levels are determined by measuring the levels of the DREAM protein or of variants thereof.

In a second step of the second method of the invention, the expression levels of the DREAM-encoding gene determined in a sample of a subject who has been diagnosed of a prodromic stage of a neurodegenerative disease are compared to a reference value, wherein a decrease in the expression levels of the DREAM-encoding gene with respect to a reference value is indicative of a prodromic stage of a neurodegenerative disease in said subject.

Determination of the reference value has been described previously. In a particular embodiment, the reference value in the context of the third method of the invention is the expression level of the gene of interest, i.e. DREAM, in a subject who has been diagnosed with a prodromic stage of a neurodegenerative disease and who has not developed any neurodegenerative disease. In another embodiment, the reference value can correspond to an average value obtained from a pool of subjects diagnosed with a prodromic stage of a neurodegenerative disease who have not developed any neurodegenerative disease. Said reference sample is typically obtained by combining equal amounts of samples from a subject population. In another embodiment, the reference value corresponds to the expression levels of DREAM-encoding gene in a sample from the subject whose diagnosis of a neurodegenerative disease is to be determined according to the third method of the invention, wherein said expression levels in said sample were determined prior to the diagnosis of the prodromic stage of a neurodegenerative disease in said subject.

In a particular embodiment of the third method of the invention, the subject diagnosed with the prodromic stage of a neurodegenerative disease is a human. Particularly, the prodromic stage of a neurodegenerative disease comprises mild cognitive impairment (MCI).

Neurodegenerative diseases predicted by the third method of the invention comprise, without being limited to, Alzheimer's disease, Huntington's disease, Parkinson's disease and neurodegeneration related to Down's syndrome. In a preferred embodiment, the neurodegenerative disease the prodromic stage of which is predicted by the method of the invention is Alzheimer's disease.

### Method for the selection of a subject for a therapy for prevention and/or treatment of a prodromic stage of a neurodegenerative disease and/or of a neurodegenerative disease (fourth method of the invention)

In a further aspect, the invention relates to an *in vitro* method for the selection of a subject for a therapy for the prevention and/or treatment of a prodromic stage of a neurodegenerative disease or a therapy for the prevention and/or treatment of a neurodegenerative disease (*fourth method of the invention*) comprising
(i) determining the expression levels of the DREAM-encoding gene in a sample from said subject, wherein said sample is not a sample of a solid tissue of the central nervous system, and
(ii) comparing the expression level obtained in (i) to a reference value,
wherein if the expression levels of the DREAM-encoding gene are decreased with respect to a reference value, then the subject is selected for said therapy.

As previously mentioned, the prodromic stage of a neurodegenerative disease comprises mild cognitive impairment (MCI). Neurodegenerative diseases in relation to the fourth method of the invention comprise Alzheimer's disease, Huntington's disease, Parkinson's disease and neurodegeneration related to Down's syndrome. In a preferred embodiment, the neurodegenerative disease is Alzheimer's disease.

In the first step of the fourth method of the invention, the expression levels of the DREAM-encoding gene are determined in a sample from a subject who is a potential candidate for a therapy for the prevention and/or treatment of a prodromic stage of a neurodegenerative disease or a therapy for the prevention and/or treatment of a neurodegenerative disease. In a particular embodiment, the subject is a human.

Methods for determining the expression levels of the DREAM-encoding gene include methods for determining the expression levels of the corresponding transcriptional product and methods for determining the expression levels of the corresponding translational product, and have been previously described in the context of the first method of the invention. In a particular embodiment, the expression levels are determined by measuring the levels of the DREAM protein or of variants thereof.

The sample from a subject for a therapy for the prevention and/or treatment of a prodromic stage of a neurodegenerative disease or a therapy for the prevention and/or treatment of a neurodegenerative disease is a sample from a solid tissue of the central nervous system, or a biofluid sample. In a particular embodiment, the sample from the subject comprises lymphocytes, more particularly T lymphocytes. Preferred samples according to the invention comprise a thymus sample and a blood sample, preferably a peripheral blood sample. In an alternative particular embodiment, the sample is a biofluid not comprising lymphocytes, particularly a serum sample.

In a particular embodiment, the subject is a human, more particularly a human suffering, or candidate to suffer, from a prodromic stage of a neurodegenerative disease or a human suffering, or candidate to suffer, a neurodegenerative disease.

In a second step of the fourth method of the invention, the expression levels of the DREAM-encoding gene, determined in a sample of a candidate subject for a therapy for the prevention and/or treatment of a prodromic stage of a neurodegenerative disease or a therapy for the prevention and/or treatment of a neurodegenerative disease, are compared to a reference value, wherein a decrease in the expression levels of the DREAM-encoding gene with respect to a reference value is indicative that the candidate subject is selected for said therapy.

Determination of the reference value has been described previously. In a particular embodiment, in the context of the fourth method of the invention for the selection of a subject for a therapy for the prevention and/or treatment of a prodromic stage of a neurodegenerative disease, the reference value is the expression level of the gene of interest, i.e. DREAM-encoding gene, in a healthy subject or in a subject not diagnosed with a prodromic stage of a neurodegenerative disease or in a subject not diagnosed with a neurodegenerative disease. Alternatively, the reference value can correspond to an average value obtained from a pool of healthy subjects, from a pool of subjects not diagnosed with a prodromic stage of a neurodegenerative disease or from a pool of subjects not diagnosed with a neurodegenerative disease. Said reference sample is typically obtained by combining equal amounts of samples from a subject population.

In another embodiment, in the context of the fourth method of the invention for the selection of a subject for a therapy for the prevention and/or treatment of a neurodegenerative disease, the reference value is the expression level of the gene of interest, i.e. DREAM-encoding gene, in a healthy subject or in a subject not diagnosed with a prodromic stage of a neurodegenerative disease or in a subject diagnosed with prodromic stage of a neurodegenerative disease who has not developed a neurodegenerative disease. As previously mentioned, the reference value can correspond to an average value obtained from a pool of healthy subjects, from a pool of subjects not diagnosed with a prodromic stage of a neurodegenerative disease or from a pool of subjects who have been diagnosed with a prodromic stage of a neurodegenerative disease who have not developed a neurodegenerative disease. Said reference sample is typically obtained by combining equal amounts of samples from a subj ect population.

Therapies for the prevention and/or treatment of a prodromic stage of a neurodegenerative disease and for the prevention and/or treatment of a neurodegenerative disease have been described previously.

### Method for determining the effectiveness of a therapy for the prevention and/or the

### treatment of a neurodegenerative disease

In yet another aspect, the present invention relates to an *in vitro* method for determining whether a therapy for the prevention and/or treatment of a prodromic stage of a neurodegenerative disease in a subject suffering from a prodromic stage of a neurodegenerative disease or a therapy for the prevention and/or treatment of a neurodegenerative disease in a subject suffering from a neurodegenerative disease is effective (*fifth method of the invention*) comprising
(i) determining the expression levels of the DREAM-encoding gene in a sample from a subject treated with said therapy, wherein said sample is not sample of a solid tissue of the central nervous system, and
(ii) comparing the expression level obtained in (i) to a reference value,
wherein if the expression levels of the DREAM-encoding gene are increased with respect to a reference value, then the therapy is effective.

In particular, the method according to the invention determines the effectiveness of a therapy for the prevention and/or treatment of a prodromic stage of a neurodegenerative disease. Alternatively, the method according to the invention determines the effectiveness of a therapy for the prevention and/or treatment of a neurodegenerative disease in a subject suffering from a neurodegenerative disease.

Therapies for the prevention of a prodromic stage of a neurodegenerative disease in a subject, for the treatment of a prodromic stage of a neurodegenerative disease in a subject suffering from said prodromic stage, for the prevention of a neurodegenerative disease in a subject, as well as for the treatment of a neurodegenerative disease in a subject suffering from said neurodegenerative disease have been mentioned above.

In a first step of the method of the invention, the expression levels of the DREAM-encoding gene are determined in a sample from a subject, particularly from a subject diagnosed of a prodromic stage of a neurodegenerative disease or from a subject diagnosed with a neurodegenerative disease. Methods for determining the expression levels of the DREAM-encoding gene have been previously described in the context of the first method of the invention and include methods for determining the expression levels of the corresponding transcriptional product and methods for determining the expression levels of the corresponding translational product. In a particular embodiment, the expression levels are determined by measuring the levels of the DREAM protein or of variants thereof.

The sample from a subject suffering, or candidate to suffer, from a prodromic stage of a neurodegenerative disease or of a subject suffering, or candidate to suffer, from a neurodegenerative disease is a sample which is not from a solid tissue of the central nervous system, or a biofluid sample. In a particular embodiment, the sample from the subject comprises lymphocytes, more particularly T lymphocytes. Preferred samples according to the invention comprise a thymus sample and a blood sample, preferably a peripheral blood sample. In an alternative particular embodiment, the sample is a biofluid not comprising lymphocytes, particularly a serum sample.

In a particular embodiment, the subject is a human, more particularly a human suffering, or candidate to suffer, from a prodromic stage of a neurodegenerative disease or a human suffering, or candidate to suffer, a neurodegenerative disease.

In a second step of the method of the invention, the expression levels of the DREAM-encoding gene determined in a sample from a subject diagnosed of a prodromic stage of a neurodegenerative disease or from a subject diagnosed with a neurodegenerative disease are compared to a reference value, wherein an increase in the expression levels of the DREAM-encoding gene with respect to a reference value is indicative that the therapy for the prevention and/or treatment of a prodromic stage of a neurodegenerative disease or the therapy for the prevention and/or treatment of a neurodegenerative disease is effective.

In a particular embodiment of the method of the invention, in the context of a therapy for the prevention of a prodromic stage of a neurodegenerative disease in a subject or a therapy for the treatment of a prodromic stage of a neurodegenerative disease in a subject suffering from said prodromic stage, the reference value is the expression level of the DREAM-encoding gene in a sample from said subject determined prior to the beginning of the therapy for the prevention and/or treatment of said prodromic stage or after the initation of the therapy but at an earlier time point.

In an alternative particular embodiment of the method of the invention, in the context of a therapy for the prevention of a neurodegenerative disease in a subject or a therapy for the treatment of a neurodegenerative disease in a subject suffering from a neurodegenerative disease, the reference value is the expression level of the DREAM-encoding gene in a sample from said subject determined prior to the beginning of the therapy for the prevention and/or treatment of the neurodegenerative disease or after the initation of the therapy but at an earlier time point.

Thus, once the reference value of DREAM-encoding gene expression is established, the expression levels of the DREAM-encoding gene expressed in a sample from a subject under therapy can be compared with this reference value, and thus be assigned a level of "increased" or "decreased". In particular, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level.

Accordingly, according to the fifth method of the invention, an increase in the expression levels of the DREAM-encoding gene with respect to a reference value is indicative that the therapy for the prevention and/or treatment of a prodromic stage of a neurodegenerative disease or the therapy for the prevention and/or treatment of a neurodegenerative disease is effective.

Therapies for the prevention and/or treatment of a prodromic stage of a neurodegenerative disease and for the prevention and/or treatment of a neurodegenerative disease have been previously mentioned.

### Therapy for use in the prevention and/or treatment of a prodromic stage of a neurodegenerative disease and/or of a neurodegenerative disease in a subject

In a further aspect, the present invention relates to an anti-neurodegenerative therapy for use in the prevention and/or treatment of a prodromic stage of a neurodegenerative disease or for use in the prevention and/or treatment of a neurodegenerative disease in a subject, wherein the expression levels of the DREAM-encoding gene in a sample of said subject are decreased with respect to a reference value and wherein said sample is not sample from a solid tissue of the central nervous system.

Alternatively, the invention relates to a method for the prevention and/or treatment of a prodromic stage of a neurodegenerative disease or for the prevention and/or treatment of a neurodegenerative disease in a subject, wherein the expression levels of the DREAM-encoding gene in a sample of said subject are decreased with respect to a reference value and wherein the sample is not a sample from solid tissue of the central nervous system.

Alternatively, the invention relates to the use of a compound for the manufacture of a medicament for the prevention and/or treatment of a prodromic stage of a neurodegenerative disease or for the prevention and/or treatment of a neurodegenerative disease in a subject, wherein the expression levels of the DREAM-encoding gene in a sample of said subject are decreased with respect to a reference value and wherein said sample is not a sample from a solid tissue of the central nervous system.

Antineurodegenerative therapies for use according to the invention in the prevention and/or treatment of a prodromic stage of a neurodegenerative disease, as well as antineurodegenerative therapies for use according to the invention in the prevention and/or treatment of a neurodegenerative disease have been mentioned above.

The invention is described below by the following examples, which must be considered as merely illustrative and in no case limiting of the scope of the present invention.

### EXAMPLES

### Example 1. DREAM levels in AD murine models

The authors of the present invention have analyzed changes in DREAM content in the thymus and the testis in AD mouse models.

A significant decrease in the DREAM protein level in the thymus from J20 and Tg2576 3-months old mice was observed (Figure 1). J20 and Tg2576 are two transgenic mouse models of AD, which overexpress the human APP gene with the Swedish and the Indiana mutations, or only the second one, respectively. These results are important because they open the possibility to assess changes of DREAM content in blood samples that could be relevant for AD early diagnosis.

It was observed no difference in the levels of DREAM protein in testis from J20 and Tg2576 3-months old mice (Figure 2). These results discard the possibility of using seminal fluid for the diagnosis of AD. However, these results are important since strengthen the specificity of the changes observed in thymus and their use for AD detection.

### Example 2. DREAM levels in human clinical samples

The detection of DREAM protein in human clinical samples was next attempted by the inventors. The presence of DREAM protein was analyzed by western blot in whole cell extracts from circulating peripheral blood lymphocytes (PBLs) obtained from human healthy volunteers. The results showed detectable amounts of DREAM protein in human PBLs (Figure 3). In this study the blood samples were from a cohort of healthy volunteers older than 70 years with no clinical symptoms of AD and no report of forgetting things (Group 0) or volunteers reporting sporadic failures to remember something but otherwise also completely normal in the clinical and neuropsychological assessment (Group 0.5). Importantly, DREAM protein levels were consistently higher in PBLs from healthy volunteers from group 0 than in PBLs from the group 0.5 (Figure 3).

These results indicate that in the absence of clinical symptoms of the disease a significant reduction in DREAM levels in PBLs occurs in the group of healthy volunteers that reported occasional failures to remember something.

## Claims

1. An *in vitro* method for predicting the appearance of a neurodegenerative disease in a subject comprising:
(i) determining the expression levels of the DREAM-encoding gene in a sample from said subject, wherein said sample is not sample from a solid tissue of the central nervous system, and
(ii) comparing the expression levels obtained in (i) to a reference value,
wherein a decrease in the expression levels of the DREAM-encoding gene with respect to a reference value is indicative of a high probability of appearance of a neurodegenerative disease in said subject.

2. An *in vitro* method for the diagnosis of the prodromic stage of a neurodegenerative disease in a subject comprising:
(i) determining the expression levels of the DREAM-encoding gene in a sample from said subject, wherein said sample is not a sample from a solid tissue of the central nervous system tissue and
(ii) comparing the expression level obtained in (i) to a reference value,
wherein a decrease in the expression levels of the DREAM-encoding gene with respect to a reference value is indicative of a prodromic stage of a neurodegenerative disease in said subject.

3. An *in vitro* method for predicting whether a subject diagnosed with a prodromic stage of a neurodegenerative disease will develop said neurodegenerative disease comprising:
(i) determining the expression levels of the DREAM-encoding gene in a sample from said subject, wherein said sample is not a sample of a solid tissue from the central nervous system, and
(ii) comparing the expression level obtained in (i) to a reference value,
wherein a decrease in the expression levels of the DREAM-encoding gene with respect to a reference value is indicative of development of the neurodegenerative disease in a subject diagnosed of a prodromic stage of a neurodegenerative disease.

4. An *in vitro* method for the selection of a subject for a therapy for the prevention and/or treatment of a prodromic stage of a neurodegenerative disease or for a therapy for the prevention and/or treatment of a neurodegenerative disease comprising
(i) determining the expression levels of the DREAM-encoding gene in a sample from said subject, wherein said sample is not a sample from a solid tissue of the central nervous system, and
(ii) comparing the expression level obtained in (i) to a reference value,
wherein if the expression levels of the DREAM-encoding gene are decreased with respect to a reference value, then the subject is selected for said therapy.

5. An *in vitro* method for determining whether a therapy for the prevention and/or treatment of a prodromic stage of a neurodegenerative disease in a subject suffering from a prodromic stage of a neurodegenerative disease or a therapy for the prevention and/or treatment of a neurodegenerative disease in a subject suffering from a neurodegenerative disease is effective comprising
(i) determining the expression levels of the DREAM-encoding gene in a sample from a subject treated with said therapy, wherein said sample is not a sample from a solid tissue of the central nervous system, and
(ii) comparing the expression level obtained in (i) to a reference value,
wherein if the expression levels of the DREAM-encoding gene are increased with respect to a reference value, then it is indicative that the therapy is effective.

6. The method according to any of claims 2 to 5, wherein the prodromic stage of the neurodegenerative disease comprises mild cognitive impairment.

7. The method according to any of the preceding claims wherein the sample is selected from the group comprising a tissue sample comprising lymphocytes, and a biofluid.

8. The method according to any of the preceding claims wherein the expression levels of DREAM-encoding gene are determined by measuring the levels of mRNA encoded by said gene or the levels of DREAM protein.

9. The method according to any of the preceding claims wherein the neurodegenerative disease is selected from the group comprising Alzheimer's disease, Huntington's disease, Parkinson's disease and neurodegeneration related to Down's syndrome.

10. A method according to any of the preceding claims, wherein the subject is a human.

11. An anti-neurodegenerative therapy for use in the prevention and/or treatment of a prodromic stage of a neurodegenerative disease or for use in the prevention and/or treatment of a neurodegenerative disease in a subject, wherein the expression levels of the DREAM-encoding gene in a sample of said subject are decreased with respect to a reference value, wherein said sample is not a sample from a solid tissue of the central nervous system.

12. The anti-neurodegenerative therapy for use according to claim 11 wherein the sample is selected from the group comprising a tissue sample comprising lymphocytes and a biofluid.

13. The anti-neurodegenerative therapy for use according to any of claims 11 or 12 wherein the expression levels of the DREAM-encoding gene are determined by measuring the levels of mRNA encoded by said gene or the levels of DREAM protein.

14. The anti-neurodegenerative therapy for use according to any of claims 11 to 13 wherein the neurodegenerative disease is selected from the group comprising Alzheimer's disease, Huntington's disease, Parkinson's disease and neurodegeneration related to Down's syndrome.

15. The anti-neurodegenerative therapy for use according to any of claims 11 to 14 wherein the subj ect is a human.
